(19) **Europäisches Patentamt**

**European Patent Office**

**Office européen des brevets**

(11) **EP 0 985 407 A1**

(12) **EUROPEAN PATENT APPLICATION**

(43) Date of publication:
**15.03.2000 Bulletin 2000/11**

(51) Int. Cl.⁷: **A61K 7/18**, A61K 7/16

(21) Application number: **99111918.1**

(22) Date of filing: **09.03.1990**

(84) Designated Contracting States:
**DE FR GB IT**

(30) Priority: **13.03.1989 US 322700**

(62) Document number(s) of the earlier application(s) in accordance with Art. 76 EPC:
**90906500.5 / 0 463 117**

(71) Applicant:
**American Dental Association Health Foundation
Chicago IL 60611 (US)**

(72) Inventors:
• **Chow, Lawrence C.**
  **Potomac, Maryland 20854 (US)**
• **Takagi, Shozo**
  **Gaithersburg, Maryland 20854 (US)**

(74) Representative:
**VOSSIUS & PARTNER
Siebertstrasse 4
81675 München (DE)**

Remarks:
This application was filed on 22 - 06 - 1999 as a divisional application to the application mentioned under INID code 62.

(54) **Fluoride containing mouth rinses, dentifrices, gels, and chewable tablets**

(57)    Previous mouthwashes and dentifrices containing fluoride contributed relatively modest amounts of additional fluoride to the tooth surfaces, and scavenged calcium from the teeth. The present invention can deposit significantly more fluoride in the mouth than previously used formulations containing comparable amounts of fluoride, without scavenging calcium from the teeth. The invention includes a method of fluoridating teeth comprising (1) mixing in an aqueous environment a first component comprising a stable, non-toxic soluble calcium salt with a second component comprising a stable, non-toxic readily hydrolyzable complex fluoride compound, resulting in hydrolysis of the fluoride compound and precipitation of calcium fluoride, followed by (2) prompt application of the mixture to tooth surfaces. The invention also contemplates mouth rinses, dentifrices, gels or pastes, and chewable tablets for application of the above-described compositions.

**Description**

## BACKGROUND OF THE INVENTION

This invention was supported in part by research grant number DE05354 to the American Dental Association Health Foundation from the National Institute of Dental Research.

Self-applied fluorides in the forms of mouth rinses and dentifrices are widely used in this country and elsewhere in the world. They have been shown to be effective in reducing tooth decay. The fluoride containing mouth rinses formulated for daily use usually contain 250 parts per million (ppm) of fluoride as sodium fluoride or stannous fluoride. The fluoride dentifrices typically contain 1000 ppm of fluoride as sodium fluoride or sodium monofluorophosphate. The cariostatic effects of both of these fluoride regimens are believed to derive from their ability to deposit fluoride on the surfaces of teeth and other tissues in the mouth. Although the deposited fluoride is labile in nature and is easily leached out, the daily application of either the rinse or dentifrice can produce and maintain an elevated level of fluoride in the mouth.

U.S. Patent No. 4,556,561 discloses solutions, gels, and substantially nonaqueous dispersions that form dicalcium phosphate dihydrate under appropriate conditions, as well as methods for their use. These compositions are useful in topically fluoridating and/or mineralizing dental tissue, such as enamel, dentin, and exposed root surfaces. The incorporated fluoride is in the form of $Ca_5(PO_4)_3F$ and is more permanently retained than $CaF_2$ and other fluoridation products.

## SUMMARY OF THE INVENTION

A constant-composition titration (CCT) technique, recently developed in our laboratory, makes it possible to measure quantitatively the minute amount of fluoride deposited on the tooth surface from a single application of fluoride containing rinse or dentifrice in vitro. By using this CCT technique (reported at Sieck, Takagi and Chow, 67 J. Dent. Res. (Special Issue) Abstract 2211 (March 1988)), we were able to determine that a 1-minute rinse with a 250 ppm fluoride rinse deposited 0.34 $\mu$g/cm$^2$ of F, and a 1-minute brushing with a 1000 ppm fluoride containing dentifrice deposited 0.25 $\mu$g/cm$^2$ of fluoride on the tooth surface. Based on the recommended quantity for the rinse (10 ml) or for the dentifrice (1 gram) per application and the total surface area of the teeth in the mouth, it is estimated that less than 0.5% of the fluoride in the rinse or the dentifrice is deposited on the teeth. Some of the fluoride is also deposited in the plaque and soft tissue surfaces, but the bulk of the fluoride contained in the rinses or dentifrices is presumably expectorated (a small fraction of fluoride is also swallowed).

A major reason for this very low yield of fluoride retention is the lack of a reaction mechanism for the fluoride in the rinse or dentifrice to precipitate out during the short application time.

In contrast to the above formulations, the present invention discloses solutions, gels, dentifrices, and chewable tablets that are useful for depositing $CaF_2$ onto the surfaces of teeth and other oral tissues. These formulations are significantly more efficacious than currently used agents containing comparable amounts of fluoride for two reasons: (1) the inventive formulations can deposit up to 15 times more fluoride, and (2) the inventive formulations do not cause any loss of tooth mineral even with frequent and protracted use.

The present invention involves a two-component system. One component contains a soluble calcium salt, and the other a complex fluoride compound. When the two components are brought in contact just prior to application to the teeth, a rapid but controlled reaction precipitates extremely fine particles of calcium fluoride continuously within the application period. The inventive system, when used in the form of mouth rinses, dentifrices, gels, or chewable tablets, can deposit significantly more fluoride in the mouth than presently used formulations containing comparable amounts of fluoride. Thus, the new formulations based on this two-component system should be significantly more efficacious than those currently in use.

Also, since none of the currently used fluoride agents contains soluble calcium in the formulation, the calcium in the $CaF_2$ formed on the tooth surfaces is derived from the tooth mineral; i.e., a small amount of the tooth mineral is dissolved with each application, and some of the dissolved calcium is reprecipitated in the form $CaF_2$. In contrast, the inventive compositions, which always contain appropriate amounts of calcium, can supply the calcium needed to form $CaF_2$. Thus, the treatment reaction does not require dissolution of the tooth mineral, and no loss of tooth mineral is expected even with frequent and protracted applications.

More specifically, in accordance with the invention, the rinse or dentifrice is generally formulated in the form of two separate phases (liquids, gels, or pastes) which will be combined just before the application. Phase A contains a soluble calcium salt, and phase B contains a complex fluoride compound. Each phase is stable for indefinite periods in the absence of the other. Shelf stability of a tablet-type product can be enhanced by protecting it from contact with moisture before use. When the two phases are combined, hydrolysis of the complex fluoride will occur which produces sufficient amounts of free fluoride to cause calcium fluoride precipitation. This in turn keeps the free fluoride concentration in the mixture sufficiently low to allow continued hydrolysis of the complex fluoride compound and precipitation of calcium fluoride. With proper concentrations of calcium and complex fluoride in the two solutions, a significant amount of calcium fluoride can be deposited on the tooth surface within a typical application time.

Thus, the present invention includes a method for fluoridating teeth comprising (1) mixing in an aqueous environment a first component comprising a stable, non-toxic soluble calcium salt with a second component comprising a stable, non-toxic readily hydrolyzable complex fluoride compound, resulting in hydrolysis of the fluoride compound and precipitation of calcium fluoride, followed by (2) prompt application of the mixture to tooth surfaces. The invention also contemplates mouth rinses, dentifrices, gels, and chewable tablets for application of the above-described compositions.

## DESCRIPTION OF THE PREFERRED EMBODIMENTS

More specifically, the present invention involves a method and materials for fluoridating teeth in situ, in a safe manner simple enough to constitute a regular part of a routine of in-home oral hygiene. The precipitation of fine calcium fluoride particles directly on the tooth surfaces accomplished by the invention is notably advantageous.

The invention contemplates use of a first component comprising a soluble calcium salt as a source of calcium. The salt and the component overall should be non-toxic enough for oral use at the intended levels on a regular basis, and stable for the desired shelf life. Examples of appropriate calcium salts include calcium chloride, calcium acetate, calcium butylate, calcium citrate, calcium lactate, calcium salicylate, and all other non-toxic salts of calcium and inorganic or organic acids which can dissolve in an aqueous solution, preferably to the extent of at least $2\times10^{-3}$ moles/L (approximately 0.008 grams of Ca in 100 grams of water).

The invention also contemplates a second component comprising a complex fluoride compound as a source of fluoride. Once again, the complex fluoride compound and the second component overall should be non-toxic enough for oral use at the intended levels on a regular basis, and stable for the desired shelf life. Note that the first and second components may be in contact, as in a chewable tablet, provided that the product is dry and thus will not permit mixing of the components in an aqueous environment leading to hydrolysis of the complex fluoride compound, until water is added. The preferred fluoride compound for purposes of this invention is sodium fluorosilicate. Other complex fluoride compounds which have hydrolytic properties similar to those of sodium fluorosilicate are within the scope of the present invention. These compounds include the fluorostannate, fluorozirconate, fluoroborate and fluorophosphate salts. Monofluorophosphate, a fluoride agent used widely in dentifrices, is also generally classified as a complex fluoride, but its hydrolysis is too slow to precipitate a significant amount of calcium fluoride within the typical 1-minute application time. Thus, monofluorophosphate and other slow hydrolyzing complex fluoride compounds are excluded from the invention.

The first component containing the calcium salt and the second component containing the complex fluoride compound are then mixed in an aqueous environment and promptly applied to the tooth surfaces. The mixing may be accomplished in various ways depending on the forms in which the inventive compositions are used. These include:

(1) For fluoride rinses, the two solutions may be contained in separate compartments in a receptacle or container or in separate receptacles or containers. Measured amounts of the two solutions are then delivered to a third compartment, preferably near the opening of a single multi-compartment receptacle (similar to the bottles used for the ACT® brand fluoride rinse). Mixing will occur as the solutions are combined in this compartment, and when the solution is swished in the mouth. The combined solution should be used within a reasonable period of time, e.g., 30 seconds, after mixing.

(2) For fluoride dentifrices, the two pastes containing the two components may be held in separate compartments in a tube or tubes similar to those used for dentifrices. Measured amounts of the two pastes will then be extruded from the tube(s) onto a tooth brush. The mixing of the two components, i.e., soluble calcium and complex fluoride, will occur as the brushing begins.

(3) For professionally applied topical fluoride gels, either of the above two forms of packaging or an alternate form of packaging may be used depending on the consistency of the gel considered most desirable. Generally, measured amounts of the two gels are dispensed from the container and mixed by stirring or spatulation. The combined gel is then placed in a tray and the tray is held tightly against the upper or lower arch of teeth for a desired period, e.g., 4 minutes.

(4) For chewable tablets, mixing will occur upon chewing and dissolution of the components in the mouth.

The basic system described above can be modified to improve its effectiveness and safety for frequent use as follows:

(1) Phase B (the fluoride phase) may also contain some phosphate so that when phases A (the calcium phase) and B are combined, the solution is always supersaturated with respect to the tooth mineral to assure that no dissolution of the tooth can occur even with frequent applications.

(2) Since hydrolysis of the complex fluoride produces free fluoride as well as hydrogen ions, to maintain the pH of the dentifrice or rinse constant above approximately 5, pH buffer may be added to either pnase A and/or B.

Given below are the compositions of two formulations, one for rinses and the other for dentifrices, which have been tested.

EXAMPLE I

Fluoride Rinses: A typical rinse formulation used in our study consists of (1) solution A which is 20 mmol/L in $CaCl_2$ and 10 mmol in sodium acetate, and (2) solution B which is 4 mmol/L in $Na_2SiF_6$, and 5 mmol/L each in $Na_2HPO_4$ and $NaH_2PO_4$. Alternatively, sodium fluorostannate may be used as the complex fluoride in this formulation. When equal volumes of A and B are mixed, the total fluoride concentration is 228 ppm, which is slightly lower than the fluoride concentrations in most fluoride rinses in use today. The hydrolysis of $SiF_6^{2-}$,

$$SiF_6^{2-} + 2H_2O \rightarrow SiO_2 + 6F^- + 4H^+,$$

is controlled by both the $F^-$ and $H^+$ concentrations in the solution. Thus, the hydrolysis does not occur appreciably in solution B because of accumulation of free $F^-$ ions, but in the combined solution the precipitation of calcium fluoride removes free $F^-$ and allows the hydrolysis to proceed. This in turn allows calcium fluoride precipitation to continue. The acetate buffer consumes most of the $H^+$ ions produced by the hydrolysis reaction so that the pH remains above 5. The phosphate present in the system assures that the solution is always supersaturated with respect to hydroxyapatite and fluorapatite so that no loss of tooth mineral occurs even with frequent applications. The above rinse is preferably used by combining 5 ml each of solutions A and B and rinsing for about 1 minute.

EXAMPLE II

Fluoride Dentifrices: Dentifrices are chemically more complex than the rinses because they contain among other things abrasive particles, detergents, and nonaqueous liquids. However, the basic principle for precipitating calcium fluoride from a 2-component system described above for the rinses can also be applied to dentifrices.

For the dentifrice formulation, the total fluoride content is higher (1000 ppm in currently used dentifrices), but the recommended quantity per application is lower (1 gram for dentifrices versus 10 mL for rinses). A typical dentifrice formulation used in our study consists of (1) paste A which contains 100 grams of a fluoride free paste with methylmethacrylate particles as the abrasives, to which 0.89 grams of $CaCl_2$ and 0.33 grams of sodium acetate are added, and (2) paste B which contains 100 grams of the same fluoride free paste to which 0.30 grams of $Na_2SiF_6$, 0.014 grams of $NaH_2PO_4 \cdot H_2O$, and 0.027 grams of $Na_2HPO_4 \cdot 7H_2O$ are added. In order for paste B to be stable for long periods, it should not contain soluble calcium compounds or calcium containing abrasive particles. Also, results from recent studies show that the inorganic abrasive particles, such as hydrated silica in Pepsodent$^®$, tend to induce $CaF_2$ precipitation to occur onto the surfaces of the abrasives. This causes a reduction of fluoride deposition on tooth surfaces. Currently, we were able to obtain more reproducible results using methylmethacrylate.

When equal amounts of pastes A and B are combined, the total fluoride content is 909 ppm, less than that of most of the fluoride dentifrices presently used, and the concentrations of other components are $[CaCl_2]$=40 mmol/L, $[NaH_2PO_4 \cdot H_2O]$=0.5 mmol/L, $[Na_2HPO_4 \cdot 7H_2O]$=0.5 mmol/L, and [Na Acetate]= 20 mmol/L. The above dentifrice is preferably used by combining 0.5 grams each of pastes A and B and brushing for about 1 minute.

The CCT technique was used to measure the fluoride deposited on tooth surfaces by the above new formulations. The results show that 5.5 and 2.2 $\mu g/cm^2$ of fluoride were deposited by the dentifrice and rinse, respectively. These values are approximately 16 and 9 times higher than the fluoride deposition from the currently used fluoride dentifrices and rinses, respectively, even though the total amounts of fluoride contained in the above rinse and dentifrice formulations are 228 and 909 ppm, respectively, which are lower than those in the fluoride rinses and dentifrices currently in use. Thus, these new formulations are likely to be significantly more effective.

The amounts of fluoride deposited on tooth surfaces by the various experimental and commercial formulations are summarized in Table 1.

Table 1

| Amount of Fluoride Deposition on Tooth Surfaces Produced by 1-minute Application with the Various Formulations | | |
| --- | --- | --- |
| Treatment | Fluoride Content in ppm | Fluoride Deposited on Tooth Surfaces, $\mu g/cm^2$ |
| ACT® Fluoride Rinse | 250 | $0.34 \pm 0.10$ |
| Calcium Fluoride Slurry | 250 | $0.28 \pm 0.05$ |
| Ammonium Fluoride Soln | 250 | $0.30 \pm 0.05$ |
| Inventive Fluoride Rinse | 230 | $5.50 \pm 0.32$ |
| Crest® Toothpaste | 1000 | $0.25 \pm 0.23$ |
| Colgate® Toothpaste | 1000 | $0.04 \pm 0.03$ |
| Inventive Fluoride Toothpaste | 910 | $2.20 \pm 0.21$ |

EXAMPLE III

Chewable fluoride supplement tablets can be formulated in a similar way. Since the tablets are packaged in a water free state, the two components described above can be safely included in same tablet; the reaction between the calcium and complex fluoride will begin after they are in contact with the saliva by virtue of the chewing action.

Other topical fluoride regimens that are being used clinically at present include: (1) mouth rinses containing 900 ppm or more of fluoride for weekly use, (2) 0.4% stannous fluoride ($SnF_2$) gels, equivalent to 970 ppm in fluoride, for daily self-applications, and (3) acidulated phosphate fluoride (APF) gels, with 1.23% fluoride, 1% $H_3PO_4$, pH 3 - 3.5, for professional tray application use.

EXAMPLE IV: A Fluoride Rinse for Weekly Use

This system consists of (1) solution A, which is 80 mmol/L in $CaCl_2$ and 40 mmol/L in sodium acetate, and (2) solution B, which is 16 mmol/L in $Na_2SiF_6$, and 20 mmol/L each in $Na_2HPO_4$ and $NaH_2PO_4$. Alternatively, calcium acetate or calcium lactate may be used as the calcium source in this formulation. Coloring and flavoring agents are also added to one or both solutions to suit consumer preferences. When equal volumes of A and B are mixed, the total fluoride concentration is 912 ppm, which is about the same as that in the fluoride rinses formulated for weekly use. The chemical reactions which will occur are basically the same as those given in EXAMPLE I, except that the concentrations of all reactants are higher. It is anticipated that the reactions will proceed rapidly and most of the fluoride in the rinse will precipitate out during the one minute application time. The above rinse is preferably used by combining 5 mL each of solutions A and B and rinsing for one minute.

EXAMPLE V: A Stannous (Tin [II]) and Fluoride Containing Gel for Self-Application

The system consists of two gels: (1) gel A is 80 mmol/L in $CaCl_2$ and 40 mmol/L in sodium acetate in water, and (2) gel B is 51 mmol/L in $SnCl_2$ and 17 mmol/L in $Na_2SiF_6$ in glycerine. ($SnF_2$ gels are usually formulated in 98% glycerine to maintain the stability of $SnF_2$.) Thickening, coloring and flavoring agents are added to one or both gels to obtain the desired consistency, and to suit consumer preferences. When equal volumes of gels A and B are combined, the tin concentration is 0.30 wt%, and the total fluoride concentration is 970 ppm. These concentrations are approximately the same as those in the 0.5 wt% $SnF_2$ gels currently in use. Thus, it may be anticipated that the inventive formulation would have approximately the same antiplaque effects as those attributable to $SnF_2$ at the same concentration. Unlike the $SnF_2$ gels, however, the inventive gel will allow the calcium fluoride to rapidly precipitate onto the surface of the teeth within the one minute application time. The above gel is preferably used by placing equal volumes of gels A and B on a tooth brush and working the gels over the surface of the teeth. After one minute, expectorate, but do not rinse.

EXAMPLE VI: An Acidic Phosphate Fluoride Gel for Professional Tray Application Use

This gel, which is intended to be used in the same way as the acidulated phosphate fluoride (APF) gel, consists of two gels: (1) gel one is 0.3 mol/L in $CaCl_2$ and 0.15 mol/L in sodium acetate in water, and (2) gel B is 0.2 mol/L in $H_2SiF_6$ (we use the acid here because the sodium and other salts are not sufficiently soluble to prepare a 0.2 mol/L solution), and 0.1 mol/L each in $(NH_4)_2HPO_4$ and $(NH_4)H_2PO_4$ in water. Thickening, coloring and flavoring agents are also added to one or both gels to obtain the desired consistency, and to suit consumer preferences. When equal amounts of the two gels are combined, the total fluoride concentration is 1.14% and the $H_3PO_4$ concentration is 0.098%; these are about the same as those in the currently used APF gels. As in all other inventive formulations, hydrolysis of $SiF_6^{2-}$ ions would produce free fluoride ions which then react with the $Ca^{2+}$ ions in the formulation to cause calcium fluoride to precipitate rapidly without dissolving any significant amount of tooth mineral. The above gel is preferably used by placing equal amounts of gels A and B in a tray and applying the mixture to the surface of the teeth for four minutes.

It should be understood that the foregoing disclosure emphasizes certain specific embodiments of the invention and that all modifications or alternatives equivalent thereto are within the spirit or scope of the invention as set forth in the appended claims.

**Claims**

1. Use of a two-component system for the preparation of a composition for fluorinating teeth, comprising

    a) a first component comprising a stable, non-toxic, soluble calcium salt and
    b) a second component comprising a stable, non-toxic, readily hydrolyzable complex fluoride compound, which upon mixing in an aqueous environment result in the hydrolysis of the fluoride compound and precipitation of calcium fluoride.

2. Use as in claim 1 wherein the soluble calcium salt is a non-toxic salt of calcium and an inorganic or organic acid which can dissolve in an aqueous solution to the extent of at least $2 \times 10^{-3}$ moles per liter.

3. Use as in claim 2 wherein the soluble calcium salt is selected from the group consisting of calcium chloride, calcium acetate, calcium butylate, calcium citrate, calcium lactate, and calcium salicylate.

4. Use as in claim 3 wherein the calcium salt is calcium chloride.

5. Use as in any one of claims 1-4 wherein the complex fluoride compound is selected from the group consisting of fluorosilicate, fluorostannate, fluorozirconate, fluoroborate, and fluorophosphate.

6. Use as in any one of claims 1-4 wherein the complex fluoride compound is sodium fluorosilicate.

7. Use as in claim 1 wherein at least one of the first component and second component is a liquid.

8. Use as in claim 1 wherein at least one of the first component and the second component is a paste.

9. Use as in claim 1 wherein at least one of the first component and the second component is a gel.

10. Use as in claim 1 wherein the first component and second component are both compounded into a chewable tablet.

11. Use as in claim 1 wherein the second component further comprises one or more phosphate compounds.

12. Use as in claim 1 wherein the first component, the second component, or both components, contain a buffer.

13. Use as in claim 1 wherein the second component further comprises stannous fluoride.

14. A packaged multi-component fluoride gel or mouth rinse product comprising at least two separate compartments wherein the first compartment contains a first component comprising a stable, non-toxic calcium salt and the second compartment contains a second component comprising a stable, non-toxic readily hydrolyzable complex fluoride compound, and wherein the compartments are configured so that the contents of the first and second compartments may be mixed upon dispensing.

15. A fluoride mouth rinse product as in claim 14 in which the concentration of the fluoride compound is greater than about 900 parts per million.

**16.** A chewable fluoride supplement tablet product comprising a stable, non-toxic calcium salt and a stable, non-toxic readily hydrolyzable complex fluoride compound in a non-aqueous carrier.

**17.** A product comprising a pair of chewable tablets packaged for simultaneous use in which the first tablet comprises a stable, non-toxic soluble calcium salt and the second tablet comprises a stable, non-toxic readily hydrolyzable complex fluoride compound.

**18.** A product as in claim 14 or 16 wherein the complex fluoride compound is sodium fluorosilicate.

**19.** A product as in claim 14 or 16 wherein the calcium salt is calcium chloride and wherein the complex fluoride compound is sodium fluorosilicate.

**20.** A product as in claim 14 or 16 wherein the second component further comprises one or more phosphate compounds.

**21.** A product as in claim 14 or 16 wherein the first component, the second component, or both components, contain a buffer.

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | FR 2 170 018 A (BLENDAX-WERKE R. SCHNEIDER & CO) 14 September 1973 (1973-09-14)<br><br>* page 3, line 31 - page 4, line 32; claims 1-3 * | 1,5, 7-11, 14-20 | A61K7/18<br>A61K7/16 |
| X | GB 1 452 125 A (PROCTER & GAMBLE) 13 October 1976 (1976-10-13)<br><br>* claims 1-6 * | 1,5, 7-11, 14-20 | |
| X | DD 249 850 A (VEB ELBE-CHEMIE) 23 September 1987 (1987-09-23) * claim 1 * | 1,5,8, 11,14 | |
| A | EP 0 263 638 A (UNILEVER) 13 April 1988 (1988-04-13) * claim 1 * | 1,14 | |
| A | FR 2 329 292 A (NATIONAL RESEARCH DEVELOPMENT CORPORATION) 27 May 1977 (1977-05-27) * claim 1 * | 1,14 | **TECHNICAL FIELDS SEARCHED** (Int.Cl.6)<br><br>A61K |

The present search report has been drawn up for all claims

| Place of search | Date of completion of the search | Examiner |
|---|---|---|
| THE HAGUE | 18 November 1999 | Voyiazoglou, D |

## ANNEX TO THE EUROPEAN SEARCH REPORT
## ON EUROPEAN PATENT APPLICATION NO.

EP 99 11 1918

This annex lists the patent family members relating to the patent documents cited in the above-mentioned European search report.
The members are as contained in the European Patent Office EDP file on
The European Patent Office is in no way liable for these particulars which are merely given for the purpose of information.

18-11-1999

| Patent document cited in search report | | Publication date | Patent family member(s) | | Publication date |
|---|---|---|---|---|---|
| FR 2170018 | A | 14-09-1973 | GB | 1408922 A | 08-10-1975 |
| | | | AT | 328089 B | 10-03-1976 |
| | | | AT | 87973 A | 15-05-1975 |
| | | | DE | 2302330 A | 16-08-1973 |
| | | | DE | 2313914 A | 25-10-1973 |
| | | | US | 4397837 A | 09-08-1983 |
| GB 1452125 | A | 13-10-1976 | AT | 873073 A | 15-06-1977 |
| | | | AU | 6104973 A | 10-04-1975 |
| | | | BE | 806060 A | 12-04-1974 |
| | | | CA | 1024449 A | 17-01-1978 |
| | | | CH | 600877 A | 30-06-1978 |
| | | | DE | 2350548 A | 25-04-1974 |
| | | | ES | 419619 A | 01-08-1976 |
| | | | FR | 2202697 A | 10-05-1974 |
| | | | IE | 38371 B | 01-03-1978 |
| | | | IT | 1053523 B | 10-10-1981 |
| | | | JP | 49094187 A | 06-09-1974 |
| | | | NL | 7314072 A | 16-04-1974 |
| | | | PH | 11647 A | 08-05-1978 |
| DD 249850 | A | 23-09-1987 | NONE | | |
| EP 263638 | A | 13-04-1988 | AT | 71282 T | 15-01-1992 |
| | | | AU | 597428 B | 31-05-1990 |
| | | | AU | 7906287 A | 14-04-1988 |
| | | | CA | 1322961 A | 12-10-1993 |
| | | | DE | 3775876 A | 20-02-1992 |
| | | | IN | 166119 A | 17-03-1990 |
| | | | JP | 6037382 B | 18-05-1994 |
| | | | JP | 63101312 A | 06-05-1988 |
| | | | PH | 25089 A | 19-02-1991 |
| | | | US | 5045305 A | 03-09-1991 |
| FR 2329292 | A | 27-05-1977 | GB | 1509977 A | 10-05-1978 |
| | | | AU | 1907076 A | 04-05-1978 |
| | | | DE | 2647870 A | 18-05-1977 |
| | | | JP | 52061236 A | 20-05-1977 |
| | | | ZA | 7606233 A | 28-09-1977 |

EPO FORM P0459

For more details about this annex : see Official Journal of the European Patent Office, No. 12/82